# FASCICULE DE BREVET EUROPEEN

(11) **EP 0 812 235 B1**
(45) Date de publication et mention de la délivrance du brevet: **11.09.2002**
(21) Numéro de dépôt: 96927088.3
(22) Date de dépôt: 25.07.1996
(51) Int. Cl.: B01J 2/00, A61K 7/00

(54) **PROCEDE DE FABRICATION DE GRANULES ET GRANULES AINSI OBTENUES**
VERFAHREN ZUR HERSTELLUNG VON GRANULATEN UND DANACH HERGESTELLTE GRANULATE
METHOD FOR MAKING GRANULES, AND RESULTING GRANULES

(30) Priorité: 27.07.1995 FR 9509390
(43) Date de publication de la demande: 17.12.1997
(73) Titulaire: Bionatec(S.A.R.L.), 06410 Biot (FR)
(72) Inventeur: ZANNINI, Gaetano, F-06410 Biot (FR); BORASCHI, Domenico, F-06410 Biot (FR); JUGE, Dominique, F-06560 Valbonne (FR); MATZA, Laurence, F-06600 Antibes (FR)
(74) Mandataire: Hautier, Jean-Louis
(86) Numéro de dépôt international: FR9601181
(87) Numéro de publication internationale: WO97004861

(56) Documents cités:
- EP-A- 0 359 230
- EP-A- 0 600 775
- WO-A-93/00991
- WO-A-94/24994
- FR-A- 2 657 255
- GB-A- 2 163 348

## Description

La présente invention a pour objet un procédé original de fabrication pour la réalisation de granules dont la taille, la couleur, la solubilité et les principes actifs peuvent varier selon le produit fini demandé : les biogranules, les grains aromatiques, les microgranules, les perles à base de pollen, les produits pour le bain. L'ensemble de ces produits sera mieux explicité par la suite.

Afin que les arômes de ces produits soient suffisamment présents, il est nécessaire d'utiliser des huiles essentielles qui sont très volatiles.

Le document FR-A-2.705.571 est relatif à une association de deux produits naturels, l'un étant pris systématiquement pour base et dans la plus importante proportion : c'est l'huile grasse d'origine végétale ou animale, l'autre est une huile essentielle aromatique extraite d'une plante : melaleuca alternifolia, qui peut être remplacée, ou associée, par une autre huile essentielle aromatique de composition biochimique équivalente ou traditionnellement connue pour ses effets thérapeutiques similaires, notamment sur des affections dentaires localisées : parulies ou toutes autres parodontopathies présentant des caractéristiques infectieuses ou inflammatoires.

Le document FR-A-2.702.654 propose un produit cosmétique permettant de combattre et diminuer le relâchement cutané au niveau de l'épiderme. Il est constitué d'un mélange actif d'huiles essentielles incorporées dans une huile végétale. Ce produit est particulièrement destiné à améliorer la texture de l'épiderme.

Ces documents ne proposent que des substances liquides qui sont plus difficiles à stocker, à conserver et à utiliser.

De plus, dans notre siècle où une mauvaise hygiène de vie est souvent liée à des habitudes alimentaires néfastes, il est nécessaire d'ingérer des produits naturels et équilibrés.

Pour cela, il est connu d'utiliser des extraits végétaux.

Le document FR-A-2.662.078 a pour objet une composition cosmétologique. La composition selon l'invention renferme les matières premières suivantes : un péloïde à usage externe appelé couramment "argile" (ou glaise), de couleur blanche, jaune, rouge ou verte, selon sa provenance et la densité des métalloïdes et minéraux qu'il contient, dans une concentration de 70 à 80 % ; des composants végétaux, présents sous forme de poudres de plantes et d'huiles essentielles de plantes, dans une concentration de 18 à 19,5 % ; des composants d'origine animale, présents sous forme de dilutions homéopathiques, dites "opothérapiques" (organothérapie), dans une concentration de 0,5 à 2 % ; dans la composition prête à l'emploi, 100 % des matières premières représentent ainsi 46 % de la formule finale, 54 % d'eau ajoutée ; les composants végétaux sont les algues marines, la vigne rouge, le bouton d'or, le chou, la préle et la reine des près, auxquelles sont ajoutées des huiles essentielles de lavande, thym ou romarin.

Le mélange entre les huiles essentielles et les extraits végétaux sont des amalgames peu homogènes. De plus, les huiles naturelles très volatiles ont tendance à s'évaporer et les arômes à disparaître.

Le document FR-A-2.657.255 décrit l'obtention de nouveaux produits cosmétiques. Ceux-ci ont la particularité de contenir les principes actifs dans des polymères qui sont eux-mêmes greffés sur des particules de silice.

Le document WO-A-93/00991 concerne un procédé de préparation de substances médicamenteuses sous forme de granules possédant une densité apparente élevée, en déposant par pulvérisation un enrobage sur des particules à noyau inerte, à l'aide d'un dispositif à enrober et à granuler par l'intermédiaire des étapes suivantes, tout d'abord constitution d'un lit fluidifié de particules en matériau à noyau inerte à l'intérieur de la chambre de traitement d'un dispositif à enrober et à granuler, en faisant circuler un courant gazeux entre le disque rotatif et la paroi intérieure de ladite chambre de traitement, ensuite constitution des granules au moyen du dépôt d'un médicament sur les particules de matériau à noyau, en les revêtant par pulvérisation avec une solution du médicament ; le procédé est caractérisé par le fait que, pendant la constitution des granules, les particules revêtues par pulvérisation sont premièrement transportées vers une zone de séchage entourant la chambre de traitement, deuxièmement transportées à travers ladite zone de séchage par un courant gazeux et troisièmement renvoyées vers la chambre de traitement. Le processus de recirculation fonctionne en continu jusqu'à la constitution de granules possédant la charge médicamenteuse souhaitée.

D'une façon générale, on peut dire que les caractéristiques qui rendent originales les granules, selon l'invention, comparées aux autres techniques d'enrobage, sont les suivantes.

Dans le cas de granules solubles, le granule est soluble dans son intégralité (noyau de départ compris) et la solubilité (vitesse et milieu) est fonction des composants (qualité et quantité) et des excipients.

Le granule permet l'intégration et donc la bonne conservation des composants volatiles comme les huiles essentielles.

A cet effet, la présente invention concerne un procédé de fabrication de granules solides destinés à la réalisation de produits à croquer, à sucer, à avaler ou à dissoudre, renfermant des substances aromatiques, alimentaires, diététiques ou à usage cosmétique qui consiste :
- à réaliser un noyau, faisant office de support, constitué d'excipients associés ou non à des substances actives, l'excipient ou principe actif est une solution par exemple alcoolique, hydro-alcoolique ou aqueuse,
- à recouvrir le noyau en trois étapes, d'au moins une couche, ces trois étapes étant réalisées pour chaque couche successive, par :
   a) enrobage du noyau par des substances actives associées ou non d'excipients,
   b) séchage de la couche, et
   c) tamisage du noyau recouvert.

Ce procédé est réalisé sous aspiration dans au moins une turbine tournante autour d'un axe pour l'enrobage.

L'axe de chaque turbine tournante est incliné d'un angle de 45 à 60° par rapport à l'horizontale.

Le procédé est réalisé à la température comprise entre 18 et 25°C, sauf pour le séchage qui est effectué à 45°C maximum dans une étuve.

Il est réalisé à une hygrométrie inférieure à 70 %.

Les granules, obtenus par l'intermédiaire du procédé ci- dessus, sont caractérisées par le fait qu'ils sont chacun constitués d'une partie centrale, formant le noyau, et d'une partie périphérique, formant une ou plusieurs couches concentriques ; chaque couche est constituée d'un composant en solution et d'un composant sous forme de poudre micronisée.

D'une part, le composant en solution constitue une solution collante dont la densité est supérieure à l'eau. D'autre part, le composant, sous forme de poudre, constitue un composant volumétrique.

Le composant en solution a une densité comprise entre 1010 et 1020 et un indice de fluidité compris entre 16 et 20.

Selon un premier mode de réalisation, la composition des granules est sensiblement de :
- 69 % de maltisorb micronisé,
- 3,5 % de bicarbonate de sodium,
- 3,5 % d'acide citrique,
- 21 % d'extrait fluide concentré de plante, et
- 3% d'huiles essentielles.

Selon un second mode de réalisation, la composition des granules est sensiblement de :
- 85 % d'un mélange de bicarbonate de soude et de citrate,
- 10 % d'huiles essentielles,
- 4 % d'émulgine, et
- 1 % de luviskol.

Quel que soit le mode de réalisation, le noyau a un diamètre inférieur à 0,01 mm, constitué de maltisorb ou d'un mélange de bicarbonate de sodium et de citrate.

Selon un mode particulier de réalisation, le noyau a un diamètre de sensiblement 2 mm constitué par des grains de pollen.

La présente invention concerne un procédé de fabrication de granules de toutes formes, tailles, etc.

L'essentiel de l'invention réside dans le fait que ce procédé est caractérisé par le fait qu'il consiste à réaliser d'une part un noyau faisant office de support, et d'autre part, à recouvrir ledit noyau d'au moins une couche.

Pour être plus précis, le noyau est toujours constitué d'excipients qui peuvent être ou non associés à des substances actives. De la même façon, chaque couche, qui va venir recouvrir ledit noyau ou une couche préalablement déposée sous-jacente, est toujours réalisée à partir de substances actives qui peuvent être associées à des excipients.

Il est donc aisé de comprendre que chaque couche qui vient d'être déposée, va ensuite subir un séchage, et enfin un tamisage du noyau recouvert par la couche séchée.

Ces trois étapes, enrobage, séchage et tamisage, sont à chaque fois réalisées pour chaque couche de produit recouvrant le noyau ou une couche sous-jacente.

Pour que les granules soient réalisés de la manière la plus homogène, on utilise une turbine en acier inoxydable qui tourne autour d'un axe, lui-même incliné d'un angle de 45 à 60° par rapport à l'horizontale.

De plus, les conditions à l'intérieur de cette turbine sont très précises ; ainsi la température est toujours comprise entre 18 et 25°C à l'exception du séchage pour lequel les granules sont mis dans une étuve à 45°C. De plus, l'hygrométrie doit obligatoirement être inférieure à 70 %, et ce, afin d'éviter tout amalgame entre tous les granules d'une même turbine.

Toute la phase de production en turbine est réalisée sous une aspiration de 2.500 m³ par heure.

En fait, les granules qui sont réalisés à l'aide du procédé sont donc constituées de façon très homogène d'une partie centrale, formant le noyau, et d'une partie périphérique, formée par une ou plusieurs couches concentriques déposées selon le procédé.

Le procédé nécessite la présence de trois composants ayant des caractéristiques précises.

Tout d'abord le noyau, selon un mode de réalisation préférentiel, est solide sous forme cristalline, et son diamètre est inférieur à 0,01 mm.

Il y a ensuite un composant excipient ou principe actif qui est en solution par exemple alcoolique, hydro-alcoolique ou aqueuse, avec une densité qui est supérieure à celle de l'eau. Ce composant constitue la solution collante.

Enfin, il y a également un composant constitué également par un excipient ou un principe actif sous forme de poudre micronisée. Ce dernier composant constitue le composant volumétrique.

Quoi qu'il en soit, il y a toujours au moins un principe actif parmi les trois composants.

Le noyau et le composant volumétrique peuvent être identiques. La densité idéale de la solution collante pour la mise en oeuvre du procédé est de 1010 pour un indice de fluidité compris entre 16 et 20. Néanmoins, on peut utiliser une densité comprise entre 1010 et 1020.

Le rapport entre la solution collante à densité idéale et la composante volumétrique est variable en fonction de la taille finale du produit recherché et de la densité de la solution collante. Mais cela reste fixe pour toute la durée du procédé de fabrication.

Selon un mode de réalisation bien particulier, la composition des granules est sensiblement de :
- 69 % de maltisorb micronisé,
- 3,5 % de bicarbonate de sodium,
- 3,5 % d'acide citrique,
- 21 % d'extrait fluide concentré de plante, et
- 3% d'huiles essentielles.

Selon un autre mode de réalisation, la composition des granules est sensiblement de :
- 85 % d'un mélange de bicarbonate de soude et de citrate,
- 10 % d'huiles essentielles,
- 4 % d'émulgine, et
- 1 % de luviskol.

Selon un autre mode de réalisation, le noyau peut être constitué par des grains de pollen agglomérés et avoir un diamètre de sensiblement deux millimètres.

L'ensemble de ces caractéristiques, qui vient d'être dévoilé, permet d'obtenir une gamme de produits variables que ce soit en forme, en fonction et en mode d'utilisation. Ainsi, on peut utiliser ces granules, soit en les dissolvant, soit en les croquant, soit en les avalant.

Ces trois cas de figures sont utilisés, par exemple, pour tout ce qui est ingestion de substances actives. Mais on peut utiliser des granules qui se dissolvent lorsqu'on veut réaliser une solution telle qu'une tisane, un thé, un bain ou une inhalation.

Les différentes applications sont, bien entendu, dues à la composition des extraits végétaux et des huiles essentielles qui sont utilisés.

Parmi les substances que l'on peut réaliser, il y a les biogranules qui sont issus d'un noyau de maltisorb cristallisé et calibré. Les extraits fluides et les huiles essentielles sont déposés par pulvérisation.

Lorsque les extraits fluides sont répartis uniformément sur tous les granules, la masse devient collante et tourne difficilement dans la turbine. L'humidité contenue dans les noyaux est absorbée par l'adjonction d'une certaine quantité de mélange, permettant ainsi la libre rotation. Il suffit, ensuite, de laisser tourner quelques minutes sous aspiration de façon à éliminer totalement l'alcool contenu dans les extraits hydro-alcooliques, par exemple. Il y a ensuite séchage et tamisage pour séparer les granules de différentes tailles.

Lorsque la première couche est cristallisée, on applique une seconde couche dans les mêmes conditions. L'opération est ensuite répétée pour déposer, par couches successives, l'intégralité des principes aromatiques. La quantité et la forme (solide ou liquide) des principes contenues dans chaque granule définit le poids moyen de chaque granule.

Un autre produit consiste en la fabrication de micro-granules. Ce n'est en fait qu'une réplique des biogranules, mais avec une taille plus petite. Ils peuvent contenir, ou non, des huiles essentielles. Le pourcentage des composants peut varier en fonction des caractéristiques de solubilité et de concentration des principes actifs demandés.

Un autre produit est constitué par les perles vitaminées. Dans ce cas, le noyau de départ est constitué par les grains de pollen naturel que l'on a déjà énumérés précédemment.

Après calibrage des grains à deux millimètres de taille, les noyaux de pollen sont mis dans la turbine et la technique de dépose des couches, telle que décrite précédemment, est utilisée.

Enfin, le dernier produit concerne les produits de bain. Dans ce cas, le noyau du départ et le composant volumétrique sont constitués par un mélange de bicarbonate de soude et d'acide citrique. La solution collante est constituée par les huiles essentielles, l'émulgine, le luviskol et le colorant.

La réalisation, bien entendu, fait également appel au procédé exposé précédemment.

## Revendications

1. Procédé de fabrication de granules solides, destinés à la réalisation de produits à croquer, à sucer, à avaler ou à dissoudre, renfermant des substances aromatiques, alimentaires, diététiques ou à usage cosmétique, consistant :
- à utiliser un noyau solide, faisant office de support,
- à recouvrir le noyau, en trois étapes, d'au moins une couche, ces trois étapes étant réalisées pour chaque couche successive, par :
a) enrobage du noyau par des substances actives associées ou non d'excipients,
b) séchage de la couche, et
c) tamisage du noyau recouvert.
**caractérisé par le fait**
**qu'**on recouvre le noyau de couches d'enrobage constituées d'une part d'une solution collante, et d'autre part d'un composant sous forme de poudre micronisée.

2. Procédé, selon la revendication 1, **caractérisé en ce**
**qu'**il est réalisé sans aspiration dans au moins une turbine tournante autour d'un axe pour l'enrobage.

3. Procédé selon l'une quelconque des revendications 1 à 2 caractérisé en ce
on utilise une solution collante dont la densité est supérieure à celle de l'eau.

4. Procédé selon l'une quelconque des revendications 1 à 3 **caractérisé en ce**
**que** le noyau faisant office de support est solide sous forme cristalline.

5. Procédé selon l'une quelconque des revendications 1 à 4 **caractérisé en ce**
**que** le noyau faisant office de support est constitué d'excipients associés ou non à des substances actives.

6. Procédé, selon l'une quelconque des revendications 1 à 5,
**caractérisé en ce**
**qu'**il est réalisé à la température comprise entre 18 et 25°C, sauf pour le séchage qui est effectué à 45°C maximum dans une étuve.

7. Procédé, selon l'une quelconque des revendications 1 à 5, **caractérisé en ce**
**qu'**il est réalisé à une hygrométrie inférieure à 70 %.

8. Granules solides, **caractérisés par le fait qu'**ils sont obtenus par l'intermédiaire du procédé selon l'une quelconque des revendications 1 à 6.

9. Granules, selon la revendication 8, **caractérisés par le fait**
**que** la composition des granules est sensiblement de :
- 69 % de maltisorb micronisé,
- 3,5 % de bicarbonate de sodium,
- 3,5 % d'acide citrique,
- 21 % d'extrait fluide concentré de plante, et 3% d'huiles essentielles.

10. Granules, selon la revendication 8, **caractérisés par le fait**
**que** la composition des granules est sensiblement de :
- 85 % d'un mélange de bicarbonate de soude et de citrate,
- 10 % d'huiles essentielles,
- 4 % d'émulgine, et
- 1 % de luviskol.

11. Granules, selon l'une quelconque des revendications 7 ou 8, **caractérisés par le fait**
**que** le noyau a un diamètre inférieur à 0,01 mm, constitué de maltisorb ou d'un mélange de bicarbonate de sodium et de citrate.

12. Granules, selon l'une quelconque des revendications 8 à 11, **caractérisés par le fait**
**que** le noyau a un diamètre de sensiblement 2 mm constitué par des grains de pollen.

## Patentansprüche

1. Herstellungsverfahren für Feststoffgranulat, bestimmt zur Herstellung von zu beißenden, zu lutschenden, zu verschluckenden oder aufzulösenden Produkten, die aromatische Stoffe, Nahrungsmittel oder Diätprodukte enthalten oder kosmetischen Zwecken dienen, darin bestehend, dass:
- ein fester Kern als Trägermaterial verwendet wird und
- dieser Kern in drei Schritten mit mindestens einer Schicht umhüllt wird, wobei diese drei Schritte bei jeder darauffolgenden Schicht zur Anwendung kommen indem,
a) der Kern mit den aktiven Substanzen mit oder ohne Exzipiens umhüllt,
b) die Schicht getrocknet und
c) die beschichteten Kerne getrocknet werden,
**gekennzeichnet dadurch, dass** der Kern mit Schichten umhüllt wird, die aus einer klebenden Lösung und einem micronisiertem Pulver bestehen.

2. Verfahren nach Anspruch 1, **gekennzeichnet dadurch, dass** es ohne Ansaugen in mindestens einer, sich zum Zweck der Beschichtung um eine Achse drehenden Turbine abläuft.

3. Verfahren nach einem der Ansprüche 1 bis 2, **gekennzeichnet dadurch, dass** eine klebende Flüssigkeit verwendet wird, deren Dichte größer ist als die des Wassers.

4. Verfahren nach einem der Ansprüche 1 bis 3, **gekennzeichnet dadurch, dass** der als Trägermaterial dienende Kern ein fester, kristalliner Körper ist.

5. Verfahren nach einem der Ansprüche 1 bis 4, **gekennzeichnet dadurch, dass** der als Trägermaterial dienende Kern aus eventuell mit aktiven Substanzen gemischtem Exzipiens, besteht.

6. Verfahren gemäss einem der Ansprüche 1 bis 5, **gekennzeichnet dadurch, dass** es bei einer Temperatur zwischen 18 und 25 °C abläuft, außer während der Trocknung, die bei maximal 45 °C in einem Trockenofen stattfindet.

7. Verfahren nach einem der Ansprüche 1 bis 5, **gekennzeichnet dadurch, dass** es bei einer Luftfeuchte unter 70 % durchgeführt wird.

8. Festes Granulat, **gekennzeichnet dadurch, dass** es mit Hilfe des Verfahrens nach einem der Ansprüche 1 bis 6 erhalten wird.

9. Granulat nach Anspruch 8, **gekennzeichnet dadurch, dass** die Zusammensetzung des Granulats in etwa die folgende ist:
- 69 % micronisiertes Maltisorb,
- 3,5 % Natriumbicarbonat,
- 3,5 % Citronensäure,
- 21 % flüssiger konzentrierter Pflanzenextrakt und
- 3 % etherische Öle.

10. Granulat nach Anspruch 8, **gekennzeichnet dadurch, dass** die Zusammensetzung des Granulats in etwa die folgende ist:
- 85 % einer Mischung aus Natriumbicarbonat und Citronensäure,
- 10 % etherische Öle,
- 4 % Emulgin,
- 1 % Luviskol.

11. Granulat nach einem der Ansprüche 7 oder 8, **gekennzeichnet dadurch, dass** der Kern einen Durchmesser kleiner 0,01 mm besitzt und aus Maltisorb oder einer Mischung aus Natriumbicarbonat und Citrat besteht.

12. Granulat nach einem der Ansprüche 8 bis 11, **gekennzeichnet dadurch, dass** der Kern einen Durchmesser von etwa 2 mm hat und aus Pollenkörnern besteht.

## Claims

1. Process for manufacturing solid pellets, for use in products to be crunched, sucked, swallowed or dissolved, containing aromatic, food, diabetic substances or for cosmetic use, consisting in:
- the use of a solid core, acting as support,
- covering the core, in three stages, with at least one layer, these three stages being executed for each successive layer by:
a) coating the core with active substances associated or not with excipients,
b) drying the layer, and
c) sifting the covered core.
**Characterized by** the fact
that the core is covered with layers of coating made up on the one hand of a sticky solution, and on the other of a component in the form of micronised powder.

2. Process according to claim 1, **characterized by** the fact
that it is carried out without aspiration in at least one turbine revolving round an axis for coating.

3. Process according to any of claims 1 to 2 **characterized by** the fact
that use is made of a sticky solution with higher density than water.

4. Process according to any of claims 1 to 3 **characterized by** the fact
that the core acting as the support is solid in crystalline form.

5. Process according to claims 1 to 4 **characterized by** the fact
that the core acting as support consists of excipients associated or not with active substances.

6. Process according to any of claims 1 to 5 **characterized by** the fact
that it is carried out at a temperature ranging between 18 and 25°C, except for drying which is carried out at a maximum of 45°C in a drying oven.

7. Process according to any of claims 1 to 5 **characterized by** the fact
that it is carried out at a hygrometry below 70%.

8. Solids pellets, **characterized by** the fact
that they are obtained by the process according to any of claims 1 to 6.

9. Pellets according to claim 8 **characterized by** the fact
that the composition of the pellets consists appreciably of:
- 69% of micronised maltisorb,
- 3.5% of sodium bicarbonate,
- 3.5% of citric acid,
- 21% of concentrated plant fluid extract, and
- 3% essential oils.

10. Pellets according to claim 8 **characterized by** the fact
that the composition of the pellets is roughly:
- 85% a mixture of citrate and sodium bicarbonate,
- 10% of essential oils,
- 4% of emulgine, and
- 1% of luviskol.

11. Pellets according to claims 7 or 8 **characterized by** the fact
that the core has a diameter less than 0.01 mm, made up of multisorb or a mixture of sodium bicarbonate and citrate.

12. Pellets according any of claims 8 to 11, **characterized by** the fact
that the core has an approximate diameter of 2mm and consists of pollen grains.
